# EUROPEAN PATENT SPECIFICATION

(11) **EP 1 414 468 B1**
(45) Date of publication and mention of the grant of the patent: **15.12.2004**
(21) Application number: 02759940.6
(22) Date of filing: 31.07.2002
(51) Int. Cl.: A61K 31/58, A61P 11/06

(54) **PHARMACEUTICAL COMPOSITION COMPRISING SALMETEROL AND BUDESONIDE FOR THE TREATMENT OF RESPIRATORY DISORDERS**
PHARMAZEUTISCHE ZUSAMMENSETZUNG ENTHALTEND SALMETEROL UND BUDESONID ZUR BEHANDLUNG VON ATEMKRANKHEITEN
COMPOSITION PHARMACEUTIQUE COMPRENANT SALMETEROL ET BUDESONIDE POUR LE TRAITEMENT DE TROUBLES RESPIRATOIRES

(30) Priority: 07.08.2001 BE 200100132
(43) Date of publication of application: 06.05.2004
(73) Proprietor: Galephar M/F, 6900 Marche-en-Famenne (BE)
(72) Inventor: VANDERBIST, Francis, B-1650 Beersel (BE); DEBOECK, Arthur, Gurabo, Puerto Rico 00778 (US); BAUDIER, Philippe, B-1180 Uccle (BE)
(74) Representative: Powis de Tenbossche, Roland
(86) International application number: PCT/BE2002/000132
(87) International publication number: WO 2003/013547

(56) References cited:
- V.MANCINI, L.PINTO: "Fluticasone propionate or budesonide with salmeterol in bronchial severe asthma in pediatric age" ALLERGY, vol. 53, no. suppl.43, 1998, page 185 XP001013216
- N.VAITKIENE E.A.: "Optimal inhaled steroids dose determination for asthma patients using monotherapy or combining it with salmeterol" EUROPEAN RESPIRATORY JOURNAL SUPPLEMENT, vol. 10, no. 25, 1997, page 105S XP001013222
- J.FERRES E.A.: "Budesonide combined with salmeterolin the treatment of asthma in children" EUROPEAN RESPIRATORY JOURNAL, vol. 12, no. suppl.29, 1998, page 69s XP001013401
- P.ODEBACK: "Is the addition of salmeterol more effective than doubling the dose of budesonide in mild asthma?" EUROPEAN RESPIRATORY JOURNAL, vol. 12, no. suppl.28, 1998, page 38s XP001008684
- G.FUGLSANG E.A.: "Effect of salmeterol treatment on nitric oxide level in exhaled air and dose-response to terbutaline in children with mild asthma" PEDIATRIC PULMONOLOGY, vol. 25, no. 5, 1998, pages 314-321, XP001073773
- D.H.YATES E.A.: "An inhaled glucocorticoid does not prevent tolerance to the bronchoprotective effect of a long-acting inhaled beta2-agonist" AMERICAN JOURNAL OF RESPIRATORY AND CRITICAL CARE MEDICINE, vol. 154, no. 6 part 1, 1996, pages 1603-1607, XP001073766
- D.H.YATES: "Effect of short- and long-acting inhaled beta2-agonists on exhaled nitric oxide in asthmatic patients" EUROPEAN RESPIRATORY JOURNAL, vol. 10, no. 7, 1997, pages 1483-1488, XP001073772
- L.P.NIELSEN E.A.: "Salmeterol reduces the need for inhaled corticosteroid in steroid-dependent asthmatics" RESPIRATORY MEDICINE, vol. 93, no. 12, 1999, pages 863-868, XP001073799

## Description

### FIELD OF THE INVENTION

The present invention relates to improvements in the treatment of asthma and other respiratory disorders. More particularly, it relates to the use of a composition comprising an effective dose of the long acting bronchodilator drug, salmeterol or a physiologically acceptable salt of salmeterol, and an effective dose of the steroidal anti-inflammatory drug budesonide or a physiologically acceptable salt of budesonide, for the treatment of respiratory disorders such as asthma and to pharmaceutical compositions containing the two active ingredients.

### BACKGROUND OF THE INVENTION

Asthma is characterized by airway inflammation that is manifested by airway hyperresponsiveness to a variety of stimuli and by airway obstruction that is reversible spontaneously or in response to treatment; reversibility may be incomplete in some patients.
Asthma is the third leading cause of preventable hospitalization in United States.
There are about 470,000 hospitalizations and more than 5,000 deaths a year from Asthma.
Asthma causes recurring episodes of coughing, wheezing, chest tightness, and difficult breathing. Asthma attacks can be life threatening. They can be prevented.
Asthma is a chronic inflammatory disorder of the airways. Chronically inflamed airways are hyperresponsive; they become obstructed and airflow is limited (by bronchoconstriction, mucus plugs, and increased inflammation) when airways are exposed to various stimuli, or triggers.
Common asthma triggers (that is factors that make asthma worse) include viral infections; allergens such as house dust mites (in bedding, carpets, and fabric-upholstered furnishings), animals with fur, cockroaches, pollens, and molds; tobacco smoke; air pollution, exercise; strong emotional expressions; chemical irritants, and drugs (such as aspirin and beta blockers).

Asthma attacks (or exacerbations) are episodic, but airway inflammation is chronically present. Asthma is a chronic disorder requiring long-term management. For many patients, this means taking preventive medication every day.
Asthma can change over time. Asthma can be mild, moderate or severe; asthma attacks can be life-threatening. The severity of asthma varies among individuals, and it can change in one individual over time. Treatment decisions are made based on the severity of asthma.
Asthma can be treated and controlled so that almost all patients can:
- prevent troublesome symptoms night and day
- prevent serious attacks
- require little or no quick-relief medication
- have productive, physically active lives
- have (near) normal lung function

Asthma may be preventable. For infants with a family history of asthma or atopy, it is highly likely that avoiding exposure to passive smoking and to house dust mites, cat and cockroach allergens will help prevent the initial development of asthma. For adults, avoiding exposure to chemical sensitizers in the workplace is helpful.

Salmeterol (F) is a selective β2 adrenoreceptor agonist which produces effective dose-proportional bronchodilation, which persists for up to 12 hours, in patients with reversible obstructive respiratory disease. Bronchodilation is significant within minutes of inhalation, maximal within 2 hours, and at therapeutic doses is equivalent to that produced by standard doses of traditional β2-agonists (salbutamol or albuterol, fenotenol, terbutaline).

Because of its long duration of action, salmeterol offers significant therapeutic advantages over shorter-acting β2-agonists in the treatment of nocturnal and exercise- induced asthma.

It has been demonstrated that inhaled salmeterol administered after or before inhaled corticosteroids was well tolerated.

Budesonide (FP) is a synthetic corticosteroid. Inhaled Budesonide at doses < or = 800 µg/day provided effective corticosteroid maintenance treatment in patients with mild to moderate asthma, in randomised, controlled clinical studies of 4 to 24 weeks in duration. Dosages of 100 to 400 µg twice daily have produced consistent improvement in spirometric measures of lung function, have reduced the frequency of as-needed β2-agonist bronchodilator use, asthma symptom scores and night-time awakenings, and have prevented asthma exacerbations compared with placebo.

Pharmaceutical compositions containing a combination of long-acting β2-agonists and corticosteroid agents are described in three patent applications. EP 416950 describes a composition containing salmeterol and beclomethasone. EP 416951 discloses a composition containing salmeterol and fluticasone. US 5,674,860 (corresponding to WO93/11773) describes a composition containing formoterol and budesonide. Not disclosed is a pharmaceutical composition comprising both salmeterol and budesonide (or salt) in a single composition and inhalable in one puff.

Several others have demonstrated that corticosteroids and long-acting β₂ mimetics, given in combination, may improve the symptoms related to asthma and/or allow to decrease the dose of corticosteroids needed (Mancini and al, Ferres and al, Nielsen and al). But all those studies were performed by combining two separate treatments, for instance, budesonide with one inhaler device and salmeterol with another inhaler device.

Consequently, some pharmaceutical companies have patented compositions comprising a corticosteroid and a β₂ mimetic in the same device or formulation, so allowing to take both products in one inhalation. This kind of combination clearly improves patient's comfort and compliance. For instance, a composition containing budesonide and formoterol (US 5,674,860) or a composition containing fluticasone and salmeterol (EP416950) have been described.

Never described was a composition allowing to administrate to the patient an effective dose of budesonide and an effective dose of salmeterol in one single inhalation. In the present invention, both molecules are indeed preferably comprised in the same galenical formulations, whereby ensuring the administration by inhalation of budesonide and salmeterol in the right ratio. When budesonide and salmeterol are present in each microparticles to be inhaled, a correct molecular ratio of salmeterol active in the lung/budesonide active in the lung can be ensured. Another advantage of such a composition containing inhaled budesonide and salmeterol in the same formulation is economical. Indeed, the present invention only requires one device while the separate administration of budesonide and salmeterol requires two separate devices, so increasing the cost of the therapy.

### BRIEF DESCRIPTION OF THE INVENTION

The invention relates to a pharmaceutical composition for inhalation, comprising as active ingredient an effective amount of salmeterol or a physiologically or therapeutically acceptable salt of salmeterol or a solvate thereof, an effective amount of budesonide or a physiologically or therapeutically acceptable salt of budesonide or a solvate thereof, and advantageously at least one pharmaceutically acceptable carrier which can be solid, partly solid, or liquid.
The composition is adapted for the substantially simultaneous, preferably simultaneous inhalation of the active ingredients with a molecular ratio salmeterol component/budesonide component in the range of 1:2 to 1:50.
The composition is advantageously in the form of a dose of active ingredient to be administered by inhalation to a patient in need, whereby the amount of salmeterol or salt or solvate (expressed in salmeterol base ) is less than about 50 µg in said dose, advantageously less than 40µg, preferably less than 35 µg and most preferably less than 30µg. The dose can be contained in a container, such as a capsule containing a monodose. The dose can also be prepared by a device adapted for taking a determined volume of composition from a multidose container, said predetermined volume corresponding to a dose or a portion of a maximum dose.
The composition of the invention is advantageously adapted for ensuring a substantially simultaneous initial action of the salmeterol and of the budesonide.

The invention relates also to a method of treatment of asthma and other inflammatory respiratory disorders which comprises administering by inhalation to humans in need of such treatment effective amounts of salmeterol or a physiologically salt of salmeterol or a solvate thereof, and budesonide or a therapeutically salt of budesonide or a solvate thereof, wherein said effective amounts are administered substantially simultaneously, preferably simultaneously, to the human in need with a molecular ratio of the salmeterol component to the budesonide component is in the range 1:2 to 1:50, together with a pharmaceutically acceptable carrier. In the method of the invention, a composition of the invention is advantageously used.

The invention further relates to a process for the preparation of a composition for treating by inhalation a patient suffering asthma or other inflammatory respiratory disorders, in which effective amounts of salmeterol or a physiologically salt of salmeterol or a solvate thereof, and budesonide or a therapeutically salt of budesonide or a solvate thereof are mixed together with a pharmaceutically acceptable carrier and are processed in a form for substantially simultaneous administration to the human in need with a molecular ratio of the salmeterol component to the budesonide component is in the range 1:2 to 1:50.

### DESCRIPTION OF THE INVENTION

The combination of a first generation β2-agonist agent (like salbutamol, fenoterol, ...) together with a corticosteroid agent, although potentially beneficial, was not possible in the past, since the duration of action of the two classes of drugs were different, respectively 4 to 6 hours for the β2-agonist and approximately 12 hours for the corticosteroid.

With the apparition of long-acting β2-agonist bronchodilators, which are active for approximately twelve hours, the combination of such agents with corticosteroids became possible, using a twice daily administration of the drug combination.

The present invention is based on the concept of a novel combination therapy whereby salmeterol (and/or a physiologically acceptable salt and/or solvate thereof) and budesonide (and/or a physiologically salt and/or solvent thereof) are administered at least substantially simultaneously, preferably simultaneously, by inhalation. The invention particularly relates to a pharmaceutical composition for inhalation containing therapeutically active amounts of salmeterol (or salt) and budesonide (or salt). The composition comprises advantageously at least one carrier. The inhaled pharmaceutical composition may be a Dry Powder Inhaler (DPI), a Metered Dose Inhaler (MDI) or a powder/solution for nebulization.

The new combination has not only a greater efficacy and duration of bronchodilator action but the combination has also a rapid onset of action.

Another significant advantage is the higher compliance of the patient since two drugs are inhaled at one time, thereby avoiding the necessity of using two different inhalers. This simplifies life considerably and makes life more comfortable and secure.

The rapid onset of action of salmeterol as a bronchodilator gives the patient immediate confirmation that he has taken an adequate dose and thereby avoiding overdosing of the β2-agonist agent and the corticosteroid.

The combination according to the present invention permits a twice daily regimen as a basic treatment of asthma and particularly allows for coverage of nocturnal asthma.

The present invention provides a medicine or drug containing a combination of a therapeutically active amount of (i) salmeterol (and/or a physiologically acceptable salt and/or solvate thereof) and (ii) budesonide (and/or a physiologically acceptable salt and/or solvate thereof).

The invention also provides a pharmaceutical composition for administration by inhalation in the treatment of respiratory disorders which comprises salmeterol (and/or a physiologically acceptable salt and/or solvate thereof) and budesonide (and/or a physiologically acceptable salt and/or solvate thereof).

The invention also relates to the manufacture of salmeterol (and/or a physiologically acceptable salt and/or solvate thereof) and budesonide (and/or a physiologically acceptable salt and/or solvate thereof) in the manufacture of a medicine for combination therapy in the treatment of respiratory disorders.

The molecular ratio of salmeterol or salmeterol containing component to budesonide or budesonide containing component is preferably within the range 1:2 to 1:50.

The intended dose regimen is a twice daily administration, where the suitable daily dose of salmeterol (expressed as salmeterol base ) is in the range 10 to 100 µg with a preferred dose of 20 to 40 µg and the suitable daily dose for budesonide is 50 to 2000 µg with a preferred dose of 100 to 800 µg.

The dose actually used will strongly depend on the patient and the severity of the disease.

The combination may be suitably inhaled from a nebulizer, from a pressurized Metered Dose Inhaler or from a Dry Powder Inhaler.

The dry powder inhaler may be either a multidose system (reservoir system) or a monodose system in which the powder is pre-packaged in either capsules (hard gelatin, HPMC or other pharmaceutically acceptable capsules) or in blisters.

When used as dry formulation, the composition of the invention is advantageously in the form of a powder with a mean particle size lower than 50µm, advantageously lower than 25µm, preferably lower than 10µm, especially lower than 5µm, such as about 3 or 4µm. For example, at least 90% by weight of the particles have a size lower than 10µm, while less than 5% by weight of the particles have a size lower than 0.1µm.
Such a dry formulation may consist of a simple mixing of particles containing one distinct active agent, such as a mix of budesonide containing particles and salmeterol containing particles. However, in specific embodiments, each active particles contain budesonide and salmeterol, preferably in the appropriate range of 1:2 to 1:50 (ratio salmeterol/budesonide).

According to a specific embodiment, the active microgranules containing both budesonide (as such, or as a salt or solvate) and salmeterol (as such, or as salt or solvate) have an average molecular ratio of salmeterol component to budesonide component. In said embodiment, at least 50% by weight, advantageously at least 70% by weight, preferably at least 90% by weight of the active microgranules have a molecular ratio of salmeterol component to budesonide component comprised between 0.5 and 1.5 times the average molecular ratio, advantageously between 0.7 and 1.3 times the average molecular ratio, preferably between 0.85 and 1.15 times the average molecular ratio.

When using dry microparticles, the amount of carrier is advantageously such that the weight ratio carrier/active ingredients is comprised between 1 and 500, advantageously between 5 and 100, preferably between 10 and 50.

The particles containing salmeterol, budesonide or the both can also be be prepared by using the process disclosed in US 6,221,398, the content of which is incorporated by reference.
The present invention is further illustrated by means of some examples.

### BRIEF DESCRIPTION OF THE FIGURE

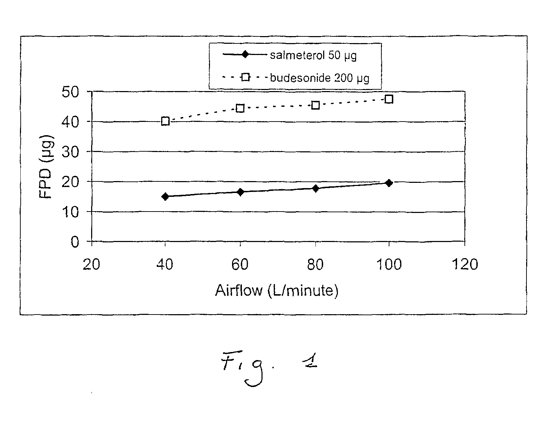

Figure 1 shows the influence of the inhalation airflow on the in vitro lung deposition (FPD) of a composition combining budesonide 200 µg and salmeterol xinafoate 76.2 µg (= 50 µg of salmeterol base) inhaled with a Miat Monodose Inhaler (n=3, 4 liters of air).

### DESCRIPTION OF EXAMPLES

In the examples, salmeterol xinafoate is used. The amount of salmeterol xinafoate used in the various formulation is expressed as salmeterol base. Formulation 1 contains thus 0.05 mg salmetrol base, formulation 2 : 0.025 mg salmeterol base, etc.

### Example 1

### Formulation 1

| *Active ingredients* | mg/capsule | mg/capsule | mg/capsule |
|---|---|---|---|
| Salmeterol (as xinafoate) | 0.050 | 0.050 | 0.050 |
| Budesonide | 0.400 | 0.200 | 0.100 |

| *Inactive ingredients* | | | |
|---|---|---|---|
| anhydrous lactose | 17.32 | 17.50 | 17.50 |
| lactose monohydrate | 7.42 | 7.50 | 7.50 |

The mix of active ingredients with lactose is filled into nr.3 hypromellose capsules. As said capsules correspond to a monodose, the patient in need knows that after inhalation, he received simultaneously salmeterol and budesonide.

### Example 2

### Formulation 2

| ***Active ingredients*** | mg/dose | mg/dose |
|---|---|---|
| Salmeterol (as xinafoate) | 0.025 | 0.025 |
| Budesonide | 0.200 | 0.400 |

| ***Inactive ingredients*** | | |
|---|---|---|
| anhydrous lactose | 25.000 | 25.000 |

The mix of active ingredients with lactose is filled in nr. 3 hard gelatine capsules.
In said composition, the lactose inactive ingredient forms a mixture of particles with a size comprised between 50 and 200µm, such as between 100 and 160µm.
As said capsules correspond to a monodose, the patient in need knows that after inhalation, he received simultaneously salmeterol and budesonide.

### Example 3

### Metered Dose Inhaler

| ***Active ingredients*** | mg/dose |
|---|---|
| Salmeterol (as xinafoate) | 0.050 |
| Budesonide | 0.200 |

| ***Inactive ingredients*** | |
|---|---|
| Propellant | 50 µl |
| Stabilizer | 0.200 |

### Example 4

| *Active ingredients* | mg/capsule |
|---|---|
| Salmeterol (as xinafoate) | 0.025 |
| Budesonide | 0.400 |

| *Inactive ingredients* | |
|---|---|
| anhydrous lactose | 17.32 |
| lactose monohydrate | 7.42 |

### Example 5 : combined particles containing salmeterol and budesonide

Combined particles of budesonide and salmeterol xinafoate have been prepared from a solution containing budesonide and salmeterol in soluble form (for example an ethanol or a chloroform solution) or from a solution in which salmeterol is soluble, but the budesonide particles are insoluble.

When using a solution containing both active ingredients in soluble form, it is often advantageous to use a mixture of solvents selected from the group comprising methanol, ethanol, water, chloroform, acetone, isopropanol, chloroform, etc.

When using a solution in which salmeterol is soluble but in which budesonide is insoluble, the salmeterol solution can be used for coating the particles of budesonide. For example an aqueous solution of salmeterol xinafoate is spray dried on budesonide particles.

The following table gives the content of various compositions comprising active particles containing salmeterol and budesonide with a mean particle size of 3 - 4 µm, and inactive particles of anhydrous lactose with a particle size of 100-160µm.

| | | | |
|---|---|---|---|
| composition | 8 | 9 | 10 |
| Budesonide/salmeterol xinafoate weight ratio for the active particles | 8 | 4 | 2 |
| Weight ratio Inactive particles/active particles | 50 | 100 | 180 |

These compositions can be placed in capsules (hard gelatin, hypromellose capsule) for the preparation of mono dose, each capsule containing an amount of salmeterol xinafoate corresponding to a dose of 25µg of salmeterol base.

### Example 6 : in vitro deposition tests

In vitro deposition tests (assessment of Fine Particle Dose) have been performed on the formulation given in example 1 (budesonide 200 µg + salmeterol 50 µg) called F1 hereinbelow. This formulation has been tested using the Miat Monodose Inhaler.
The recommendations of the European Pharmacopoeia (4^{th} Ed., 2002, 2.9.18.) concerning the way to perform the in-vitro testing for DPIs are now well established.
The in vitro deposition results performed on the Multistage Liquid Impinger (MLI) (E.P. 4^{th} Ed., 2002, 2.9.18 Apparatus C).

The results of Fine particle dose (FPD) is the dose in µg of particles having a diameter inferior to 5 µm, Mass Median aerodynamic diameter (MMAD) and Geometric standard deviation (GSD) obtained are given in the table herebelow. The values of FPD is considered to be directly proportional to the amount of drugs able to reach the pulmonary tract in vivo. Consequently the lower the values of FPD the lower,, the estimated lung deposition.
In order to make a comparative assessment of the composition making the object of the present invention, the in vitro deposition test has also been performed on a marketed form of budesonide (Pulmicort® Turbuhaler® 200 µg, Astra Zeneca) and a marketed form of salmeterol xinafoate (Serevent® Diskus® 50 µg, Glaxo Smithkline). Each device has been tested at the airflow defined by the European Pharmacopoeial test i.e. 60 Liters / minute for the Pulmicort® Turbuhaler® , 70 liters / minute for the Serevent® Diskus® and 100 L/ minute for the monodose Miat inhaler used for administering the present invention. The volume of air inhaled through the apparatus was 4 liters for each device.

**Table 1 :**

| Assessment of Fine Particle Dose (n=3) | | | | |
|---|---|---|---|---|
| Parameter | F1 (budesonide 200 µg + salmeterol 50 µg) Miat Monodose Inhaler | | Pulmicort® Turbuhaler® 200 µg | Serevent® Diskus® 50 µg |
| | salmeterol | budesonide | budesonide | salmeterol |
| FPD (ug / dose) | 18.1 ± 0.87 | 47.6 ± 4.2 | 45.2 ± 3.5 | 7.89 ± 0.53 |
| MMAD | 3.23 ± 0.06 | 3.76 ± 0.12 | 3.95 ± 0.09 | 4.02 ± 0.09 |
| GSD | 1.72±0.1 | 1.74 ± 0.08 | 1.62 ± 0.11 | 1.48 ± 0.08 |

As it can be observed, the DPI composition containing a combination of budesonide 200 µg and salmeterol 50 µg, presents a similar FPD for budesonide than the reference (Pulmicort® Turbuhaler® 200 µg, Astra Zeneca ) and a more than twice as high value of FPD for salmeterol than the reference (Serevent® Diskus® 50 µg, Glaxo Smithkline).

### Example 7 : In vitro deposition in function of the airflow

As the present invention is primarily destinated to patients with asthma and / or bronchopneumopathy chronic obstructive (BPCO) i.e. patients with relatively weak lung functions, it was of interest to assess the dependence of the FPD to the airflow. Indeed, moderate or severely ill patients, children and elderly people present lower lung functions and are therefore unable to inhale at the airflow recommended by the European Pharmacopoeia. The lung deposition of the present invention has therefore been assessed at different airflow i.e. 40, 60, 80 and 100 L / minute). The results obtained are given in the figure 1 attached to the present specification, said figure showing the influence of the inhalation airflow on the in vitro lung deposition (FPD) of a composition combining budesonide 200 µg and salmeterol 50 µg inhaled with a Miat Monodose Inhaler (n=3, 4 liters of air).

This figure shows that, the ratio inhalable budesonide ( FDP budesonide in µg) / inhalable salmeterol (FDP salmeterol in µg) is substantially independent from the air flow, said ratio being about 2.3 - 2.4.

As shown in figure 1, the FPD of budesonide and salmeterol is very lowly influenced by the value of the airflow so insuring that even patients with low lung functions will be able to inhale the drugs properly and hence to obtain a therapeutical dose in the lungs.

## Claims

1. A dry powder pharmaceutical composition for inhalation, comprising as active ingredient effective amounts of salmeterol or a physiologically salt of salmeterol or a solvate thereof, and budesonide or a therapeutically salt of budesonide or a solvate thereof, together with at least one pharmaceutically acceptable carrier which comprises anhydrous lactose, wherein the composition is in the form of a dose of active ingredient to be administered by inhalation to a patient in such a need, whereby the amount of salmeterol or physiologically salt of salmeterol or a solvate thereof expressed in salmeterol base, is less than 50 µg in said dose, and in which the composition is adapted for the substantially simultaneous inhalation of active ingredient with a molecular ratio of salmeterol component to budesonide component in the range 1:2 to 1:50.

2. The pharmaceutical composition according to claim 1, in the form of a dose of active ingredient to be administered by inhalation to a patient in need, whereby the amount of salmeterol or physiologically salt of salmeterol or a solvate thereof, expressed in salmeterol base, is less than 40 µg in said dose, preferably less than 35 µg and most preferably less than 30 µg.

3. The pharmaceutical composition according to claim 1, wherein the weight ratio carrier/active ingredients is comprised between 1 and 500, advantageously between 5 and 100, preferably between 10 and 30.

4. The pharmaceutical composition according to claim 3, wherein the formulation further contains lactose monohydrate or another pharmaceutical sugar as pharmaceutically acceptable excipient.

5. The pharmaceutical composition according to claim 1, wherein said composition comprises active microgranules comprising salmeterol or a physiologically salt of salmeterol or a solvate thereof, budesonide or a therapeutically salt of budesonide or a solvate thereof, and advantageously at least one carrier, said active microgranules having a size of less than 10 µm, preferably less than 5 µm.

6. The pharmaceutical composition according to claim 5 wherein the molecular ratio of salmeterol component to budesonide component of the active microgranules is in the range 1:2 to 1:50.

7. The pharmaceutical composition according to claim 6, wherein microgranules containing the active ingredients have an average molecular ratio of salmeterol component to budesonide component and wherein at least 50% by weight, advantageously at least 70% by weight, preferably at least 90% by weight of the active microgranules have a molecular ratio of salmeterol component to budesonide component comprised between 0.5 and 1.5 times the average molecular ratio, advantageously between 0.7 and 1.3 times the average molecular ratio, preferably between 0.85 and 1.15 times the average molecular ratio.

8. The pharmaceutical composition according to claim 5, wherein the microgranules containing the active ingredients comprises as carrier , a mixture of anhydrous lactose and lactose monohydrate .

9. The pharmaceutical composition according to claim 1, wherein a monodose of the dry powder formulation is filled into a pharmaceutically acceptable capsule for administration with a monodose dry powder inhaler device.

10. The pharmaceutical composition according to claim 1, wherein a monodose of the dry powder formulation is filled into a pharmaceutically acceptable hypromellose capsule.

11. The pharmaceutical composition of claim 1, wherein the composition is in a dry form for administration using a multi-dose inhalation device.

12. The pharmaceutical composition according to claim 1, wherein the salmeterol component is in the form of xinafoste.

13. The pharmaceutical composition of claim 1 wherein the salmeterol component and the budesonide component are in a form adapted for simultaneous administration, advantageously for simultaneous initial action after administration.

14. The pharmaceutical composition of claim 1, in which the carrier is a mixture of anhydrous lactose with lactose monohydrate, with a weight ratio of about 17.5/7.5.

15. A process for the preparation of a composition according to anyone of the claims 1 to 14.

## Patentansprüche

1. Pharmazeutische Trockenpulverzusammensetzung zur Inhalation, enthaltend, als Wirkstoff, wirksame Mengen von Salmeterol oder eines physiologischen Salzes von Salmeterol oder eines Solvates davon und Budesonid oder eines therapeutischen Salzes von Budesonid oder eines Solvates davon, zusammen mit wenigstens einem pharmazeutisch unbedenklichen Träger, der wasserfreie Lactose enthält, wobei die Zusammensetzung in Form einer durch Inhalation an einen dessen bedürftigen Patienten zu verabreichenden Wirkstoffdosis vorliegt, wobei die Menge an Salmeterol bzw. des physiologischen Salzes von Salmeterol bzw. eines Solvates davon, ausgedrückt als Salmeterolbase, in der Dosis weniger als 50 µg beträgt, und wobei die Zusammensetzung für die im wesentlichen gleichzeitige Wirkstoffinhalation mit einem molekularen Verhältnis von Salmeterolkomponente zu Budesonidkomponente im Bereich von 1:2 bis 1:50 ausgelegt ist.

2. Pharmazeutische Zusammensetzung nach Anspruch 1 in Form einer einem bedürftigen Patienten durch Inhalation zu verabreichenden Wirkstoffdosis, wobei die Menge an Salmeterol bzw. physiologischem Salz von Salmeterol bzw. einem Solvat davon, ausgedrückt als Salmeterolbase, in der Dosis weniger als 40 µg, vorzugsweise weniger als 35 µg und besonders bevorzugt weniger als 30 µg beträgt.

3. Pharmazeutische Zusammensetzung nach Anspruch 1, wobei das Gewichtsverhältnis von Träger zu Wirkstoffen zwischen 1 und 500, vorteilhafterweise zwischen 5 und 100, vorzugsweise zwischen 10 und 30, liegt.

4. Pharmazeutische Zusammensetzung nach Anspruch 3, wobei die Formulierung weiterhin Lactose-monohydrat oder einen anderen pharmazeutischen Zucker als pharmazeutisch unbedenklichen Hilfsstoff enthält.

5. Pharmazeutische Zusammensetzung nach Anspruch 1, wobei die Zusammensetzung aus einem aktiven Mikrogranulat besteht, das Salmeterol oder ein physiologisches Salz von Salmeterol oder ein Solvat davon, Budesonid oder ein therapeutisches Salz von Budesonid oder ein Solvat davon und vorteilhafterweise mindestens einen Träger enthält, wobei das aktive Mikrogranulat eine Größe von weniger als 10 µm, vorzugsweise weniger als 5 µm, aufweist.

6. Pharmazeutische Zusammensetzung nach Anspruch 5, wobei das molekulare Verhältnis der Salmeterolkomponente zur Budesonidkomponente des aktiven Mikrogranulats im Bereich von 1:2 bis 1:50 liegt.

7. Pharmazeutische Zusammensetzung nach Anspruch 6, wobei das die Wirkstoffe enthaltende Mikrogranulat ein durchschnittliches molekulares Verhältnis von Salmeterolkomponente zu Budesonidkomponente hat und wobei wenigstens 50 Gew.-%, vorteilhafterweise wenigstens 70 Gew.-%, vorzugsweise wenigstens 90 Gew.-% des aktiven Mikrogranulats ein molekulares Verhältnis von Salmeterolkomponente zu Budesonidkomponente aufweisen, das zwischen dem 0,5- und 1,5fachen des durchschnittlichen molekularen Verhältnisses, vorteilhafterweise zwischen dem 0,7- und 1,3fachen des durchschnittlichen molekularen Verhältnisses, bevorzugt zwischen dem 0,85- und 1,15fachen des durchschnittlichen molekularen Verhältnisses liegt.

8. Pharmazeutische Zusammensetzung nach Anspruch 5, wobei das die Wirkstoffe enthaltende Mikrogranulat als Träger eine Mischung von wasserfreier Lactose und Lactose-Monohydrat enthält.

9. Pharmazeutische Zusammensetzung nach Anspruch 1, wobei eine einzelne Dosis der Trockenpulverformulierung zur Verabreichung mit einem Einzeldosis-Trockenpulverinhaliergerät in eine pharmazeutisch unbedenkliche Kapsel gefüllt wird.

10. Pharmazeutische Zusammensetzung nach Anspruch 1, wobei eine einzelne Dosis der Trockenpulverformulierung in eine pharmazeutisch unbedenkliche Hypromellosekapsel gefüllt wird.

11. Pharmazeutische Zusammensetzung nach Anspruch 1, wobei die Zusammensetzung in trockener Form für die Verabreichung mit einem Mehrfachdosisinhalationsgerät vorliegt.

12. Pharmazeutische Zusammensetzung nach Anspruch 1, wobei die Salmeterolkomponente in Form von Xinafoat vorliegt.

13. Pharmazeutische Zusammensetzung nach Anspruch 1, wobei die Salmeterolkomponente und die Budesonidkomponente in einer für die gleichzeitige Verabreichung, vorteilhafterweise für die gleichzeitige Initialwirkung nach der Verabreichung, ausgelegten Form vorliegen.

14. Pharmazeutische Zusammensetzung nach Anspruch 1, wobei es sich bei dem Träger um eine Mischung aus wasserfreier Lactose mit Lactose-Monohydrat mit einem Gewichtsverhältnis von etwa 17,5/7,5 handelt.

15. Verfahren zur Herstellung einer Zusammensetzung nach einem der Ansprüche 1 bis 14.

## Revendications

1. Composition pharmaceutique de poudre sèche pour l'inhalation, comprenant comme ingrédient actif des quantités efficaces de salmétérol ou d'un sel physiologiquement acceptable de salmétérol ou d'un solvate de celui-ci, et de budésonide ou d'un sel thérapeutiquement acceptable de budésonide ou d'un solvate de celui-ci, conjointement avec au moins un support pharmaceutiquement acceptable qui comprend du lactose anhydre, dans laquelle la composition est sous la forme d'une dose d'ingrédient actif à administrer par inhalation à un patient qui en a besoin, par quoi la quantité de salmétérol ou de sel physiologiquement acceptable de salmétérol ou d'un solvate de celui-ci exprimée en base de salmétérol, est inférieure à 50 µg dans ladite dose, et où la composition est adaptée à l'inhalation substantiellement simultanée de l'ingrédient actif avec un rapport moléculaire du composant de salmétérol au composant de budésonide dans l'intervalle de 1:2 à 1:50.

2. Composition pharmaceutique selon la revendication 1, sous la forme d'une dose d'ingrédient actif à administrer par inhalation à un patient qui en a besoin, par quoi la quantité de salmétérol ou de sel physiologiquement acceptable de salmétérol ou d'un solvant de celui-ci, exprimée en base de salmétérol, est inférieure à 40 µg dans ladite dose, de préférence inférieure à 35 µg et davantage préféré inférieure à 30 µg.

3. Composition pharmaceutique selon la revendication 1, dans laquelle le rapport en poids support/ingrédients actifs est compris entre 1 et 500, avantageusement entre 5 et 100, de préférence entre 10 et 30.

4. Composition pharmaceutique selon la revendication 3, dans laquelle la formulation contient en outre du monohydrate de lactose ou un autre sucre pharmaceutique comme excipient pharmaceutiquement acceptable.

5. Composition pharmaceutique selon la revendication 1, dans laquelle ladite composition comprend des microgranules actifs comprenant du salmétérol ou un sel physiologiquement acceptable de salmétérol ou un solvate de celui-ci, du budésonide ou un sel thérapeutique acceptable de budésonide ou un solvate de celui-ci, et avantageusement au moins un support, lesdits microgranules actifs ayant une taille inférieure à 10 µm, de préférence inférieure à 5 µm.

6. Composition pharmaceutique selon la revendication 5, dans laquelle le rapport moléculaire du composant de salmétérol au composant de budésonide des microgranules actifs est dans l'intervalle de 1:2 à 1:50.

7. Composition pharmaceutique selon la revendication 6, dans laquelle les microgranules contenant les ingrédients actifs ont un rapport moléculaire moyen du composant de salmétérol au composant de budésonide et dans laquelle au moins 50% en poids, avantageusement au moins 70% en poids, de préférence au moins 90% en poids des microgranules actifs ont un rapport moléculaire du composant de salmétérol au composant de budésonide compris entre 0,5 et 1,5 fois le rapport moléculaire moyen, avantageusement entre 0,7 et 1,3 fois le rapport moléculaire moyen, de préférence entre 0,85 et 1,15 fois le rapport moléculaire moyen.

8. Composition pharmaceutique selon la revendication 5, dans laquelle les microgranules contenant les ingrédients actifs comprennent comme support, un mélange de lactose anhydre et de monohydrate de lactose.

9. Composition pharmaceutique selon la revendication 1, dans laquelle une monodose de la formulation de poudre sèche est introduite dans une capsule pharmaceutiquement acceptable pour l'administration avec un dispositif d'inhalation de poudre sèche en monodose.

10. Composition pharmaceutique selon la revendication 1, dans laquelle une monodose de la formulation de poudre sèche est introduite dans une capsule d'hypromellose pharmaceutiquement acceptable.

11. Composition pharmaceutique selon la revendication 1, dans laquelle la composition est sous une forme sèche pour l'administration utilisant un dispositif d'inhalation multidose.

12. Composition pharmaceutique selon la revendication 1, dans laquelle le composant de salmétérol est sous la forme de xinafoate.

13. Composition pharmaceutique selon la revendication 1, dans laquelle le composant de salmétérol et le composant de budésonide sont sous une forme adaptée pour l'administration simultanée, avantageusement pour l'action initiale simultanée après administration.

14. Composition pharmaceutique selon la revendication 1, dans laquelle le support est un mélange de lactose anhydre avec du monohydrate de lactose, avec un rapport en poids de 17,5/7,5.

15. Procédé pour la préparation d'une composition selon l'une quelconque des revendications 1 à 14.
